Europäisches Patentamt

European Patent Office

Office européen des brevets

(11) Publication number: **0 375 810**
**A1**

(12) # EUROPEAN PATENT APPLICATION

(21) Application number: **88203036.4**

(22) Date of filing: **29.12.88**

(51) Int. Cl.⁵: **A61F 13/02, A61L 15/00**

(43) Date of publication of application:
**04.07.90 Bulletin 90/27**

(84) Designated Contracting States:
**AT BE CH DE ES FR GB GR IT LI LU NL SE**

(71) Applicant: **Fischer-Schreiner, Cornelia Gesina Maria**
**1, W. Bilderdijklaan**
**NL-1401 AP Bussum(NL)**

(72) Inventor: **Fischer-Schreiner, Cornelia Gesina Maria**
**1, W. Bilderdijklaan**
**NL-1401 AP Bussum(NL)**

(74) Representative: **Keijser, Johannes Maurits L.F., Mr. et al**
**EXTERPATENT B.V. P.O. Box 90649**
**NL-2509 LP The Hague(NL)**

(54) **Adhesive plaster and bandage for medical purposes, as well as methods of manufacturing them.**

(57) The invention relates to plasters and bandages, and more generally to all self-adhesive and non-adhesive products for medical purposes, intended to be applied to and to remain visible for shorter or longer time periods on the human skin. The material, or at least the surface thereof which, in use, will be visible, is coloured and the colour has been chosen from the group comprising brown and yellow in all shades and from light to dark, black, and optical white. In methods of manufacturing the products, one may either use coloured material or colouring treatment, the colour of which has been chosen from the group mentioned above.

EP 0 375 810 A1

## Adhesive plaster and bandage for medical purposes, as well as methods of manufacturing them.

The invention relates to a self-adhesive strip for medical purposes, intended to be applied to and to remain visible for shorter or longer time periods on the human skin - such as adhesive plaster, plaster with gauze cushion, tacky swathing-band, heat therapy plaster, and all other specific products comprised under the notion "self-adhesive strip for medical purposes", particularly those defined in the specification - comprising a carrier strip provided with a self-adhesive layer on one side.

The invention furthermore relates to non-adhesive product for medical purposes intended to be applied to and to remain visible for shorter or longer time periods on the human skin, such as bandage, gauze, swathing-band, heat therapy bandage, compression bandage, net bandages, stocking bandage, attachment clip, face mask, examination or surgery glove and all other specific products comprised under the notion "non-adhesive product for medical purposes", particularly those as defined in the specification.

Products of this nature for medical purposes are invariably of what is called "skin colour". Much research has been done in the past to develop materials, dyes, inks or toplayers which are said to resemble skin colour best. Equally invariably this is understood to be the skin colour of so-called white people. Non-white people have no other option than to accept these products for medical purposes as are in the market on an exclusive basis, and the colour of which is not akin to their own skin colour.

Not only can this be considered to be discriminating, but it is believed that by the presence of pigment in the skin, as a result of a very long development of peoples in certain regions of the earth, the people are better adapted to the natural circumstances in such region. Especially when plasters and bandages would have to be carried for a longer time period and to the extent that they are of bigger dimensions, "white" products can be considered to disturb the adaptation to the natural circumstances; this might adversely affect the overall health situation while the use of the products is primarily intended to be for the benefit.

Therefore, the invention proposes that, in case of the adhesive products mentioned above, the material of the carrier strip is a coloured material, or the carrier strip, at the surface opposite the surface which is provided with the adhesive layer, is provided with a coloured layer, in either case the colour having been chosen from the group comprising brown and yellow in all shades and from light to dark, black, and optical white.

The colours brown, yellow and black are meant to cover all skin colours of people other than those which are traditionally called whites. Based upon the same inventive idea of abandoning the exclusivity of adaptation to the skin colour of what is only a minority of humankind, optical white is proposed, because non-white people might now prefer to use plasters in a colour contrasting with their own skin colour without this being the skin colour of the so-called white people. As is known, this latter colour, although it is called "white" is in fact more like pink. Anyhow, in connection with the present invention, the term "optical white" is used as defining physical white other than skin-colour-white as a shade of pink.

The self-adhesive layer may be of substantially the same colour as the coloured carrier or the coloured layer on the carrier.

As regards bandage and other non-adhesive products as defined above in the second paragraph, the invention proposes, in line with what was said above with regard to the adhesive products, that the material of the product is again a coloured material, or at least at one surface is provided with a coloured layer, the colour of which has been chosen from the group comprising brown and yellow in all shades and from light to dark, black, and optical white.

The invention claims exclusive rights not only for the products, on the basis of claims 1-5, but also for the method of manufacturing these products, on the basis of claims 6-9. The method claims will be fully understood after what was said above in connection with the products and will therefore not be quoted at this place.

The invention is applicable to all adhesive and non-adhesive products for medical purposes which are intended to be applied to and to remain visible for shorter or longer time periods on the human skin. In order to make clear that there are no limitations within this generic definition, the following is pointed out.

As far as the self-adhesive products are concerned, the carrier may be of the more or less traditional fabric material, but also the more modern plastic materials of various types fibre fleece which is used because it is porous, silk and stretch material which is elastic in one or two directions.

The adhesive material may be correspondingly coloured. The plasters may be provided with gauze cushions, whether or not as vulnerary plasters or with impregnated gauze cushions.

They may be special purpose plasters such as vaccination plaster, injection plaster, fingertip plaster, cornplaster or callosity plaster, sporttape, fixation tape. This group may further comprise swathing-band and heat therapy plaster in the tacky or adhesive variation. They may be provided, in the usual manner, at greater length, so that parts can be cut with a size desired for each case, or they may be units, ready for use.

As regards the bandages of non-adhesie type, also all material appropriate for the purpose envisaged can be used. This groups comprises roller bandage for general use, dressing, bandage for special use such as knee-, ankle-, elbow- or wristbandage; tennisarm and tenniselbow bandage, sport bandage, kneestockings and support stockings, and the product known as racket-grip. Furthermore heat-therapy materials such as knee, shoulder and elbow warmers, heat belts. Then there are swathing-band, fixation bandage, support bandage, compress and compression bandage and the special purpose bandage such as elastic knitting and stocking bandage and anti-thrombosis stockings.

Other special products are face masks, surgery gloves or examination gloves, and attachment clips for bandage.

As regards the manner of producing the colour of, at least, one surface of the product which will be visible during use - it is not always unambiguous when this side would be called the top or the outside and therefore these terms will be avoided - there is basically a choice between material which is or can be coloured in itself, or to provide at least one layer of the appropriate colour.

Providing such a coloured layer can be done by dyeing or printing,or by providing a special surface layer or foil, but the invention does not exclude any other method of providing colour to the product.

It is also to be noted that, in addition to the use of coloured material or the application of a colouring treat ment according to the invention, any other customary treatment may be applied, such as the provision of a coating which make pores or other apertures in the product closed or filled, surface treatment as a basis for proper attachment of ink which is provided by printing, or the provision of a surface coating which will prevent attachment of the adhesive from the other side, or a treatment which will make the material waterresistant.

The invention does not exclude that, in combination with the materials which will be visible and which will therefore be coloured, gauzes or dressing bandages be used in their natural, generally optical white colour, as far as these materials are destined to come into contact with wounds, because the optical white colour thereof will better allow the medical staff to medically examine the wound, and the course of the healing process by looking at the gauze or dressing when it is removed.

Finally it is pointed out that coloured strip material, including particularly self-adhesive strip material, is known already. The colour range thereof does also encompass black, optical white, brown and yellow. These known strips are for example anti-static tape and marking tape. None of them is adapted to be used for medical purposes, as is the essential idea of the present invention.

**Claims**

1. Self-adhesive strip for medical purposes, intended to be applied to and to remain visible for shorter or longer time periods on the human skin - such as adhesive plaster, plaster with gauze cushion, tacky swathing-band, heat therapy plaster, and all other specific products comprised under the notion "self-adhesive strip for medical purposes", particularly those defined in the specification - comprising a carrier strip provided with a self-adhesive layer on one side, **characterized in that** the material of the carrier strip is a coloured material, the colour of which has been chosen from the group comprising brown and yellow in all shades and from light to dark, black, and optical white.

2. Self-adhesive strip for medical purposes, intended to be applied to and to remain visible for shorter or longer time periods on the human skin - such as adhesive plaster, plaster with gauze cushion, tacky swathing-band, heat therapy plaster, and all other specific products comprised under the notion "self-adhesive strip for medical purposes", particularly those defined in the specification - comprising a carrier strip provided with a self-adhesive layer on one side, **characterized in that** the carrier strip, at the surface opposite the surface which is provided with the adhesive layer, is provided with a coloured layer, the colour of which has been chosen from the group comprising brown and yellow in all shades and from light to dark, black, and optical white.

3. The product as claimed in claim 1 or 2, **characterized in that** the self-adhesive layer is of substantially the same colour as the coloured carrier or the coloured layer on the carrier.

4. Non-adhesive product for medical purposes intended to be applied to and to remain visible for shorter or longer time periods on the human skin, such as bandage, gauze, swathing-band, heat therapy bandage, compression bandage, net bandages, stocking bandage, attachment clip, face mask, examination or surgery glove and all other specific products comprised under the notion "non-adhesive product for medical purposes", particularly those as defined in the specification, **characterized in that** the material of the product is a coloured material, the colour of which has been chosen from the group comprising brown and yellow in all shades and from light to dark, black, and optical white.

5. Non-adhesive product for medical purposes intended to be applied to and to remain visible for shorter or longer time periods on the human skin, such as bandage, gauze, swathing-band, heat therapy bandage, compression bandage, net bandages, stocking bandage, attachment clip, face mask, examination or surgery glove and all other specific products comprised under the notion "non-adhesive product for medical purposes", particularly those as defined in the specification, **characterized in that** the product, at least at one surface, is provided with a coloured layer the colour of which has been chosen from the group comprising brown and yellow in all shades and from light to dark, black, and optical white.

6. Method of manufacturing a self-adhesive strip for medical purposes, intended to be applied to and to remain visible for shorter or longer time periods on the human skin - such as adhesive plaster, plaster with gauze cushion, tacky swathing-band, heat therapy plaster, and all other specific products comprised under the notion "self-adhesive strip for medical purposes", particularly those defined in the specification - comprising a carrier strip provided with a self-adhesive layer on one side, **characterized in that** the strip is prepared from a carrier of coloured material, the colour of which has been chosen from the group comprising brown and yellow in all shades and from light to dark, black, and optical white.

7. Method of manufacturing a self-adhesive strip for medical purposes, intended to be applied to and to remain visible for shorter or longer time periods on the human skin - such as adhesive plaster, plaster with gauze cushion, tacky swathing-band, heat therapy plaster, and all other specific products comprised under the notion "self-adhesive strip for medical purposes", particularly those defined in the specification - comprising a carrier strip provided with a self-adhesive layer on one side, **characterized in that** the carrier material is subjected - either before or after the step of providing it with an adhesive layer - to a colouring treatment, the colour of which has been chosen from the group comprising brown and yellow in all shades and from light to dark, black, and optical white.

8. Method of manufacturing a non-adhesive product for medical purposes intended to be applied to and to remain visible for shorter or longer time periods on the human skin, such as bandage, gauze, swathing-band, heat therapy bandage, compression bandage, net bandages, stocking bandage, attachment clip, face mask, examination or surgery glove and all other specific products comprised under the notion "non-adhesive product for medical purposes", particularly those as defined in the specification, **characterized in that** the product is manufactured from a coloured material, the colour of which has been chosen from the group comprising brown and yellow in all shades and from light to dark, black, and optical white.

9. Method of manufacturing a non-adhesive product for medical purposes intended to be applied to and to remain visible for shorter or longer time periods on the human skin, such as bandage, gauze, swathing-band, heat therapy bandage, compression bandage, net bandages, stocking bandage, attachment clip, face mask, examination or surgery glove and all other specific products comprised under the notion "non-adhesive product for medical purposes", particularly those as defined in the specification, **characterized in that** the material of the product is subjected to a colouring treatment, the colour of which has been chosen from the group comprising brown and yellow in all shades and from light to dark, black, and optical white.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-3 687 136 (D.B. CARMODY) <br> * Column 1, lines 11-14 * <br> --- | 1-9 | A 61 F 13/02 <br> A 61 L 15/00 |
| Y | US-A-2 905 174 (C.W. SMITH) <br> * Column 2, lines 23-26 * <br> --- | 1-9 | |
| Y | US-A-4 161 176 (F.E. HARRIS) <br> * Claims 1,8 * <br> ----- | 1-9 | |

TECHNICAL FIELDS SEARCHED (Int. Cl.5)

A 61 F

The present search report has been drawn up for all claims

| Place of search | Date of completion of the search | Examiner |
|---|---|---|
| THE HAGUE | 17-08-1989 | PELTRE CHR. |

EPO FORM 1503 03.82 (P0401)